# EUROPEAN PATENT SPECIFICATION

(11) **EP 0 526 511 B1**
(45) Date of publication and mention of the grant of the patent: **28.05.1997**
(21) Application number: 91908153.9
(22) Date of filing: 26.03.1991
(51) Int. Cl.: A61K 38/00

(54) **METHOD AND COMPOSITION FOR TREATMENT OF CENTRAL NERVOUS SYSTEMS DISEASE STATES ASSOCIATED WITH ABNORMAL AMYLOID BETA PROTEIN**
VERFAHREN UND ZUSAMMENSETZUNG ZUR BEHANDLUNG VON ERKRANKUNGEN DES ZNS HERVORGERUFEN DURCH ABNORMALES BETA-AMYLOID-PROTEIN
PROCEDE ET COMPOSITION SERVANT A TRAITER DES ETATS PATHOLOGIQUES DU SYSTEME NERVEUX CENTRAL ASSOCIES A LA PROTEINE BETA AMYLOIDE ANORMALE

(30) Priority: 27.04.1990 US 514021; 16.10.1990 US 598383
(43) Date of publication of application: 10.02.1993
(73) Proprietor: MCMICHAEL, John, Delanson, NY 12053 (US)
(72) Inventor: KLINE, Ellis, L., Pendleton, NC 29670 (US); MCMICHAEL, John, Delanson, NY 12053 (US)
(74) Representative: Brown, John David
(86) International application number: US9101898
(87) International publication number: WO9116819

(56) References cited:
- WO-A-88/03951
- WO-A-90/14841
- US-A- 4 666 829
- US-A- 4 816 416
- US-A- 4 912 206
- SCIENCE, vol. 243, 17 March 1989, pages 1488-1490; WHITSON, JANET S. ET AL.: 'AMYLOID BETA PROTEIN ENHANCES THE SURVIVAL OF HIPPOCAMPAL NEURONS IN VITRO'
- PROCEEDINGS OF THE NATIONAL ACADEMY OF SCIENCES OF THE USA, vol. 85, no. 8, April 1988, pages 2790-2794; ALLSOP D. ET AL.: 'IMMUNOHISTOCHEMICAL EVIDENCE FOR THE DERIVATION OF A PEPTIDE LIGAND FROM THE AMYLOID BETA-PROTEIN PRECURSOR OF ALZHEIMER DISEASE'
- SCIENCE, vol. 250, 12 October 1990, pages 279-282; YANKNER B.A. ET AL.: 'NEUROTROPHIC AND NEUROTOXIC EFFECTS OF AMYLOID BETA PROTEIN REVERSAL BY TACHYKININ NEUROPEPTIDES'

## Description

### SUMMARY OF THE INVENTION

In accordance with a first aspect of the invention, there is provided a pharmaceutical composition comprising a vehicle for a single administration of amyloid beta protein or fraction thereof which comprises an amount of up to 10⁻² mg amyloid beta protein or fraction thereof and pharmaceutically inert ingredients. The total amount of the pharmaceutical and the vehicle per dosage unit should be at least 0.05 to 2cc total aqueous dosage for an aqueous form or at least about 100 mg. for a solid form so that the total amount is sufficient for convenient administration. In a preferred aspect the pharmaceutical composition has an amount of from 10⁻⁸ to 10⁻² mg. amyloid beta protein or fraction thereof. In one embodiment, the vehicle is an aqueous solution that is contained within an inert container, for example, the solution may be an injectable form. In an alternate embodiment, the pharmaceutical composition has a vehicle which is solid, such as a sublingual tablet. In another variation, the composition in the form of a suppository.

In a second aspect of the invention, there is provided an amyloid beta protein or an active fraction thereof for use in alleviating the symptoms of disease states associated with abnormal accumulation of and/or molecular organisation of amyloid beta protein, neuritic plaques or amyloid plaques associated with central nervous system and histopathologically related disorders, wherein the amyloid beta protein or the active fraction thereof is in an amount of up to 10⁻² mg. per dosage unit.

In a further aspect of the present invention, there is provided use of an amyloid beta protein or an active fraction thereof for the manufacture of a medicament for the alleviation of disease states associated with abnormal accumulation of and/or molecular organisation of amyloid beta protein, neuritic plaques or amyloid plaques associated with central nervous system and histopathologically related disorders, wherein the amyloid beta protein or the active fraction thereof is in an amount of up to 10⁻² mg. per dosage unit.

In one embodiment, the amyloid beta protein is the entire amyloid beta protein. In an alternate embodiment, the amyloid beta protein is a fraction of amyloid beta protein. In a preferred embodiment, the amount of either the amyloid beta protein or fraction thereof is about 10⁻⁴ mg. Preferably, the daily adult dosage does not exceed about 10⁻² mg. per adult bodyweight, and still more preferably does not exceed about 10⁻⁴ mg.

Cellular skeletal systems have three distinct ultrastructures made of fibrous macromolecules: microtubules, intermediate filaments and microfilaments, all of which are associated with the central nervous system (CNS) and other histopathological disorders. The neuronal intermediate filaments, defined as neurofilaments (containing amyloid beta protein constructs), are distinct from other intermediate filaments found in the cells of the central nervous system. R. D. Goldman, A. Milstead, J. A. Schloss and M. J. Yerna, Annu. Rev. Physiol. 41 p. 703-722 (1979); R. J. Lasak, Neucrosci. Res. Program Bull. 19 p. 7-32 (1981); R. J. Lasek and M. L. Shelanski, Neucrosci. Res. Program Bull. 19 p. 3-153 (1981); C. A. Maretta, ed., Neurofilaments (1983); M. L. Shelanksi and R. K. H. Liem, J. Neurochem. 33 p. 5-13 (1979). Neurofilaments are composed of three proteins with molecular weights of 200,000, 150,000 and 70,000 daltons. B. H. Toh, L. J. Gibbs, Jr., D. C. Gajdusek, J. Goudsmit and D. Dahl, Proc. Natl. Acad. Sci. USA. An additional 62,000 dalton protein is also affiliated with the above mentioned proteins. These above proteins are associated with slow axoplasmic transport. P.N. Hoffman and R. J. Lasek, J. Cell Biology 66 p. 351-366 (1975).

Alzheimer's disease, and other amyloid associated maladies such as senile dementia, Down's syndrome, Pick's disease, progressive supranuclear palsy, multiple sclerosis and others, are characterized by the presence of one or more fused fibrils of repetitive amyloid beta proteins or other similar amyloid residues such as paired helical filaments, neurofibrillary tangles, neuritic plaques, amyloid plaques and cerebrovascular amyloidosis. B.H. Anderson, D. Breinberg and M.J. Downes, Nature 298 p. 84-86 (1982). These paired helical filaments are indistinguishable immunologically and chemically from normal neurofilaments and share many of the same proteinaceous epitopes. B.H. Anderson, D. Breinberg and M.J. Downes, Nature 298 p. 84-86 (1982); B.H. Toh, L.J. Gibbs, D.C. Gajdusek, J. Goudsmit and D. Dahl, Proc. Natl. Acad. Sci. USA; K. Iqbal, I. Grundke-Iqbal, H. M. Wisnieski and R.D Terry, Brain Res. 142 p. 321-332 (1975). It has been suggested that these interfere with axonal transport. P.N. Hoffman and R.J. Lasek, J. Cell Biol. 66 p. 351-366 (1975); J.W. Griffin, P.N. Hoffman, A.W. Clark, P.T. Carroll and D.L. Price, Science 202 p. 633-665 (1978).

Using the cDNA clone of the gene encoding amyloid beta protein as a genetic probe, it was shown that the gene is located on chromosome twenty-one and is expressed in many tissues of the body. D. Goldjaber, M.I. Lerman, O.W. McBridge, U. Suffiotti, and D.C. Gaidusak, Science 235 P. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, Science 235 p. 880-884 (1987). Quantitation of amyloid beta protein expression, as seen by its mRNA levels using the cDNA probe, has revealed that its level of expression in brain tissue of Alzheimer's was not above that seen for other tissues outside the central nervous system. This was of interest to researchers when noting that amyloid plaque formation only occurs in the brain. R.E. Tanzi, J.F. Gusella, P.C. Watkins, G.A.P. Bruns, P. St.George, M.L. Vankeuren, D. Patterson, S. Pagan, D.M. Kurnit and R.L. Neve, Science 235 p. 880-884 (1987).

Amyloid beta protein is obtained through conventional means known in the art and has been characterized by various scientists. A.S. Cohen and E. Calkins, Nature 183 p. 1202 (1959), A.S. Cohen and E. Calkins, J. Cell Biology 21 p. 481 (1964); A.S. Cohen, E. Calkins and C. Levens, Am. J. Pathol. 35 p. 979 (1959). More recent work is manifested by D. Caspi, M.C. Baltz and M.K. Pepys, Mol. Biol. Med. 3 pp. 387-407 (1986); and D. Caspi, M.C. Baltz and M.K. Pepys, Mol. Biol. Med. 3 pp. 409-424 (1986). Amyloid beta protein exists in various structural forms. The amyloid beta protein that has been experimentally used and as referred to herein in terms of any specific embodiments constitutes a mixture of such forms. It is to be understood that within the scope of the present invention it is contemplated that any of the various forms of amyloid beta protein may be used.

Amyloid beta protein from the brain has been cDNA cloned and shown to contain a unique twenty amino acid NH₂-terminal sequence. Glenner, G.G. and Wong, W., Biochem. Biophys. Res. Comm. 122 No. 3, pp. 1131-35 (1984) (herein: Glenner & Wong); D. Caspi, M.C. Baltz and M.K. Pepys, Mol. Biol. Med. 3 pp. 409-424 (1986); Goldgaber, D., Lerman, M.I., McBridge, O.W., Saffiotti, U., and Gaidusak, D.C., Science 235, pp. 777-80 (1987). It is to be understood that any active fraction of beta amyloid protein that may be determined to possess the pharmaceutical efficacy expressed herein is specifically contemplated in lieu of the various structural conformations. There are various well known fractions of beta amyloid protein that are set forth in the literature including the previously discussed reference of Glenner & Wong. Subsequent to the present invention herein there has been a recognition by others that such fractions are useful in the treatment of CNS disorders such as Alzheimer's disease, including an identification of various fractions and equivalents. See Bruce A. Yankner, Lawrence K. Duffy and Daniel A. Kirschner, Science 250, pp. 279-282, 280 (1990) ("Yankner, Duffy & Kirschner").

It has been observed that a buildup of abnormally organized amyloid beta protein in brain tissue is manifested in Alzheimer's disease. See Dennis J. Selkoe and Carmela R. Abraham, "Isolation of Paired Helical Filaments and Amyloid Fibers from Human Brain", 134 Methods in Immunology 388-404 (1986). The fact that there is an accumulation of beta amyloid protein in the brain in Alzheimer patients has been demonstrated by post mortem analysis of brain tissue that manifest a concentration of amyloid beta protein as part of an accumulation of parallel filaments or neural fibrillatory tangles in the brain that appear characteristic of Alzheimer victims, along with neuritic plaque and cerebral vasculatory amyloidosis.

The presence of amyloid beta protein in fibrils and plaques in Alzheimer's disease, as well as other CNS disorders, has been suggested to be a result of a degradation product of the normal neurofilaments, D. Goldjaber, M.I. Lerman, O.W. McBridge, U. Suffiotti and D.C. Gaidusak, Science 235 p. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, Science 235 p. 880-884 (1987); M. Baudry, B.R. Dubrin, L. Beasley, M. Leon and G. Lynch, Neurobiol. Aging 7 p. 255-260 (1986); G.G. Glenner, Arch. Path. Lab. Med. 107 p. 218-282 (1983); or possibly due to improper metabolism of byproducts. Further breakdown products of amyloid beta proteins from neurofilaments have also been observed in amyloid plaques, along meningeal vascular walls, and intracortical blood vessels. S. Bahmanyar, E.J. Williams, F.B. Johnson, S. Young and D.C. Gaidusak, J. Comp. Path. 95 p. 1-5 (1985); M.E. Bruce and H. Fraser, Neuropathol. Appl. Neurobiol 1 p. 189-207 (1981); M.E. Bruce and H. Fraser, Neuropathol. Appl. Neurobiol. 7 p. 289-298 (1981); G.G. Glenner and W. Wong, J. Quaranta and G.G. Glenner, Pro. Nat. Acad. Sci. 82 p. 8729 (1985); D.J. Selkoe, C.R. Abraham, M.B. Podlisky and L.K. Duffy, J. Neurochem. 46 p. 1820 (1986).

During the mid-1960's, Solomon & Moos speculated that there is a close integration between immunological function, the central nervous system, psychophysiological factors (emotions), and disease, both physical and mental. G.F. Solomon and R.H. Moos, Arch. Gen. Psychiatry 11 p. 657-674 (1964). The integration of these systems was initially suggested through observing the presence of abnormal immunoglobulins in schizophrenic patients. G.F. Solomon and R.H. Moos, Arch. Gen. Psychiatry 11 p. 657-674 (1964); J.G. Knight, Lancet 82 p. 1073-1076 (1982); W.J. Fessel and M. Hirata-Hibi, Arch. Gen. Psychiatry 9 p. 601-613. These immune aberrations (termed autoantibodies), which seemed to target certain body cellular structures, G.F. Solomon, Psychoneuroimmunology p. 259-278 (1985); G.F. Solomon and R.H. Moos, Psychosom. Mod. 27 p. 135-149 (1981), supported the concept that there is a close communication between the CNS and the immune system. For instance, met-enkephalin is a neurotransmitter for the CNS system and is a product of activated T-helper cells. G. Zurawaki, M. Benedik, D.J. Kamb, J.S. Abrams, S.M. Zurawaki and F.O. Lee, Science 232 p. 772-775 (1986).

The appearance of autoantibodies specific to the CNS neurofilaments in patients with Alzheimer's and other CNS disorders suggests that the body's immune system may play a role in the disease process. S. Bahmanyar, R.K.H. Liem, J.W. Griffin and D.C. Gajdusek, J. Neuropathol. Exp. Neurol. 53 p. 85-90 (1984); S. Bahmanyar, M.C. Moreau-Dubois, P. Brown, F. Catala and D.C. Gajdusek, J. Neuroimmunol. 5 p. 191-196 (1983); T.S. Elizan, J. Casals and M.D. Yahr, J. Neurol. Sci, 59 p. 341-347 (1983). The autoantibodies against normal CNS neurofilaments react with the paired helical filaments in neurofibrillary tangles of Alzheimer's disease. D. Dahl and A. Bignami, Exp. Neurol. 58 p. 74-80 (1978); M.E. Bruce, J. Neuropathol. Exp. Neurol. 37 p. 595, abstract (1978).

Animal models for these CNS disorders which are induced with aluminum chloride or β,β'-iminodipropionitrite (IDPN) to form paired helical filaments in neurofibrillary tangles, also react with antibodies directed against CNS neurofilaments. J.W. Griffen, P.N. Hoffman, A.W. Clark P.T. Carroll and D.L. Price, Science 202 p. 633-665 (1978).

Control of such autoimmune reactions theoretically could lead to the alleviation of symptoms manifested by such reactions. Over the past two decades, a body of clinical literature has accumulated relating to the treatment of autoimmune disease (or, more appropriately, diseases reflecting immune dysfunction) using a technique called provocative-neutralization therapy. Miller, Annals of Allergy 38 p. 185-191 (1977); Miller, Trans. Am. Soc. Opth. & Otolar. Allergy 14 p. 159-168 (1974); Miller, Clinical Medicine 81 p. 16-19 (1974). In short, this method, which is commonly employed for allergy therapy, involves subcutaneous or sublingual introduction of an antigen known, or suspected, to provoke symptoms reflective of immune dysregulation. By serial titration of the provoking material, a concentration of that agent can often be determined which will neutralize those symptoms induced by the same substance at a different concentration. This then is a prime example of a dose-dependent phenomenon in which one dose induces a positive reaction while another dose of the same agent induces a negative response.

Although it is thought that neutralization occurs as a consequence of reestablishing homeostatic functional levels of T8 suppressor cells, it is quite possible that the same antigen used at a neutralizing concentration to reverse immune dysregulation could also, or instead, trigger endocrine and/or neuronal control mechanisms to reverse symptoms. Because of the intimate association between the three control systems (endocrine, immune, nervous) and proven communication pathways between and among the cells comprising these respective systems, a single active molecule, such as amyloid beta protein in the Alzheimer's victim, and related CNS disorders, could reverse symptoms via any or all of these routes.

In accordance with the invention, there is provided a method to stimulate the appropriate metabolic regulatory systems (immune, CNS or endocrine) which retard the progress of the symptoms of Alzheimer's and in theory should be applicable to related disease states. Observations by scientists have now indicated that the apparent elevated amyloid beta protein concentration in Alzheimer's may not be due to an increase in genomic expression, but possibly an activation of a mechanism that induces the reorganization of amyloid moieties from normal neurofilaments into paired helical filaments resulting in neurofibrillary tangles, neuritic plaques or amyloid plaques. D. Goldjaber, M.I. Lerman, O.W. McBridge, U. Suffiotti and D.C. Gaidusak, Science 235 p. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, Science 235 p. 880-884 (1987); M. Baudry, B.R. Dubrin, L. Beasley, M. Leon and G. Lynch, Neurobiol. Aging 7 p. 255-260 (1986); G.G. Glenner, Arch. Path. Lab. Med. 107 p. 218-282 (1983). The mechanisms of the present invention may result in triggering control processes that correct the rearrangement of neurofilaments, alter abnormal amyloid beta protein formation, and/or allow for clearing of the axonal transport interfering molecular structures.

The alleviation of Alzheimer's disease symptoms observed following parenterally administered amyloid beta protein as described herein likely reflects stimulation of appropriate metabolic regulatory systems in the Alzheimer's disease patients such that accumulation and/or formation of the paired helical filaments in neurofibrillary tangle, neuritic plaque and/or amyloid plaque developments are significantly altered or slowed and accumulated proteins are eliminated. This reprogramming to establish proper homeostasis would allow more efficient transmission of nerve impulses which would result in clinical improvement of treated Alzheimer's patients.

Results from early clinical work using parenteral administration of amyloid beta protein or a derivation thereof offers hope for the successful treatment of Alzheimer's disease as well as other disorders associated with amyloidosis of the central nervous system. Whether the action of the amyloid beta protein in relieving symptoms of Alzheimer's disease is due to the regulation of one or more subpopulations of T-cells, or by some other mechanism, is not known. It is also not known if a subunit of the natural, or possibly a modified amyloid beta protein, can also act to alleviate Alzheimer's disease symptoms, or if a structurally similar molecule from another source can prove effectual. It is anticipated that similar or equivalent variations would be employed by those familiar with the art.

Typically, a pharmaceutical dosage unit of the present invention for the delivery of amyloid beta protein in a low concentration comprises a liquid or solid carrier and an effective amount of amyloid beta protein. The aforesaid effective amount is preferably from about 10⁻⁸ to about 10⁻² mg., and still more preferably about 10⁻⁴ mg., amyloid beta protein in said dosage unit in association with pharmaceutically acceptable excipients. The dosage unit as used herein refers to the amount of the amyloid beta protein that is administered at one time to the patient. It is to be understood that this dosage unit may be delivered several times per day so that the daily dosage may be in multiples of up to five times the dosage unit. The same order of magnitude of daily dosage is therefore still retained for the effective amount as for the dosage unit. The upper level of the daily dosage should not exceed about 10⁻² mg. per adult.

The amyloid beta protein is administered through standard methods, including sublingual, subcutaneous and transdermal routes, and in dosage units that are either liquid or solid.

One explanation for the mode of action of this invention may be that the amount of this protein administered is sufficient to trigger a negative feedback mechanism to the body such that production of additional amyloid beta protein, possibly through breakdown of normal neurofilaments, is inhibited. Under this theory, the low level of amyloid beta protein, or a derivative thereof, gives a signal to the body to correct the abnormal synthesis/degradation process. The body sensors are then adjusted to normal metabolic control of amyloid beta protein processing that allows the proper balance to reestablish itself, alleviating the abnormal processing. The immune system, as well as the endocrine and CNS control systems, could play an integral regulatory role in response to the low dose therapy, with the amyloid protein functioning through mechanisms that not only correct the molecular organization of the amyloid beta protein moieties, but clear the interfering amyloid molecular constructs.

In a preferred embodiment, the present invention provides administration of amyloid beta protein or a derivative thereof. The amyloid beta protein may be provided either as part of a liquid solution or in a solid powder matrix, and may be administered with conventional excipients to permit ease of administration and accurate dosage delivery.

### EXAMPLE I

A 67 year old white male with a history of Alzheimer's disease for four years prior to initiating therapy presented with an inability to answer questions, to place names with faces, and to complete his sentences. His wife noted a consistent downhill progression of his condition on a monthly basis. His initial score on the objective test was 5 of a possible 30. After five months of therapy by administering four times per day the dosage unit this patient scored a 12½, was reading roads signs while travelling, and was communicating with family members. Also, the patient appeared to be more relaxed and better able to respond to his wife's efforts to assist him.

### EXAMPLE II

An 81 year old white male presented who was unable to dress himself, had a flat affect, was poorly communicative, and scored 10½ on the objective test. After three months by administering four times per day the dosage unit of Example I, he scored 17 points, was more animated in speech, could dress himself most days, and was more confident in physical actions.

### EXAMPLE III

When initiating this treatment, this patient was a 62 year old white female suffering from a fulminating form of Alzheimer's that had seen her go from being director of nursing in a chronic care establishment to, within one year, requiring constant care, unable to communicate, did not appear to recognize anyone, and scored a 1.5 on the objective test. After three months of administering four times per day the dosage unit of Example I, the patient's husband reported that warmth had returned to the patient's hands, no deterioration of any type was evident although prior to therapy he could note weekly declines, communication remained difficult but improved, she showed increased alertness, and she was not only able to recognize individuals consistently, but also was able at times to participate in conversations, and her test score rose to 7.75.

## Claims

1. A pharmaceutical composition comprising a vehicle for a single administration of amyloid beta protein or fraction thereof which comprises an amount of up to 10⁻² mg amyloid beta protein or fraction thereof and pharmaceutically inert ingredients.

2. A pharmaceutical composition according to Claim 1, wherein said amount is from 10⁻⁸ to 10⁻² mg amyloid beta protein or fraction thereof.

3. A pharmaceutical composition according to Claim 2, wherein said vehicle is an aqueous solution that is contained within an inert container.

4. A pharmaceutical composition, according to Claim 2, wherein said vehicle is a solid.

5. A pharmaceutical composition according to Claim 4, in the form of a sublingual tablet.

6. A pharmaceutical composition according to Claim 4, in the form of a suppository.

7. An amyloid beta protein or an active fraction thereof for use in alleviating the symptoms of disease states associated with abnormal accumulation of and/or molecular organisation of amyloid beta protein, neuritic plaques or amyloid plaques associated with central nervous system and histopathologically related disorders, wherein the amyloid beta protein or the active fraction thereof is in an amount of up to 10⁻² mg. per dosage unit.

8. An amyloid beta protein or fraction thereof according to Claim 7, wherein the amyloid beta protein is in the form of the amyloid beta protein itself.

9. An amyloid beta protein or fraction thereof according to Claim 7, wherein the amyloid beta protein is a fraction of amyloid beta protein.

10. An amyloid beta protein or fraction thereof according to Claim 7, wherein said amount is about 10⁻⁴ mg.

11. Use of an amyloid beta protein or an active fraction thereof for the manufacture of a medicament for the alleviation of disease states associated with abnormal accumulation of and/or molecular organisation of amyloid beta protein, neuritic plaques or amyloid plaques associated with central nervous system and histopathologically related disorders, wherein the amyloid beta protein or the active fraction thereof is in an amount of up to 10⁻² mg. per dosage unit.

12. A use according to Claim 11, wherein the amyloid beta protein is in the form of the amyloid beta protein itself.

13. A use according to Claim 11, wherein the amyloid beta protein is a fraction of amyloid beta protein.

14. A use according to Claim 11, wherein said amount is about 10⁻⁴ mg.

## Patentansprüche

1. Eine pharmazeutische Zusammensetzung, die ein Vehikel für eine Einzelverabreichung von Amyloid-Beta-Protein oder einer Fraktion desselben, das eine Menge von bis zu 10⁻² mg Amyloid-Beta-Protein oder einer Fraktion desselben umfaßt, und pharmazeutisch inerte Inhaltsstoffe umfaßt.

2. Eine pharmazeutische Zusammensetzung nach Anspruch 1, wobei besagte Menge von 10⁻⁸ bis 10⁻² mg Amyloid-Beta-Protein oder einer Fraktion desselben beträgt.

3. Eine pharmazeutische Zusammensetzung nach Anspruch 2, wobei besagtes Vehikel eine wäßrige Lösung ist, die in einem inerten Behälter enthalten ist.

4. Eine pharmazeutische Zusammensetzung nach Anspruch 2, wobei besagtes Vehikel ein Feststoff ist.

5. Eine pharmazeutische Zusammensetzung nach Anspruch 4, in der Form einer Sublingualtablette.

6. Eine pharmazeutische Zusammensetzung nach Anspruch 4, in der Form eines Suppositoriums.

7. Ein Amyloid-Beta-Protein oder eine aktive Fraktion desselben zur Verwendung bei der Linderung der Symptome von Erkrankungszuständen, assoziiert mit abnormaler Akkumulation von und/oder molekularer Organisation von Amyloid-Beta-Protein, neuritischen Plaques oder Amyloid-Plaques, assoziiert mit dem zentralen Nervensystem, und histopathologisch verwandten Störungen, wobei das Amyloid-Beta-Protein oder die aktive Fraktion desselben in einer Menge von bis zu 10⁻² mg pro Dosiseinheit vorliegt.

8. Ein Amyloid-Beta-Protein oder eine Fraktion desselben nach Anspruch 7, wobei das Amyloid-Beta-Protein in der Form des Amyloid-Beta-Proteins selbst vorliegt.

9. Ein Amyloid-Beta-Protein oder eine Fraktion desselben nach Anspruch 7, wobei das Amyloid-Beta-Protein eine Fraktion von Amyloid-Beta-Protein ist.

10. Ein Amyloid-Beta-Protein oder eine Fraktion desselben nach Anspruch 7, wobei besagte Menge etwa 10⁻⁴ mg beträgt.

11. Verwendung eines Amyloid-Beta-Proteins oder einer aktiven Fraktion desselben zur Herstellung eines Medikaments zur Linderung von Erkrankungszuständen, assoziiert mit abnormaler Akkumulation von und/oder molekularer Organisation von Amyloid-Beta-Protein, neuritischen Plaques oder Amyloid-Plaques, assoziiert mit dem zentralen Nervensystem, und histopathologisch verwandten Störungen, wobei das Amyloid-Beta-Protein oder die aktive Fraktion desselben in einer Menge von bis zu 10⁻² mg pro Dosiseinheit vorliegt.

12. Eine Verwendung nach Anspruch 11, wobei das Amyloid-Beta-Protein in der Form des Amyloid-Beta-Proteins selbst vorliegt.

13. Eine Verwendung nach Anspruch 11, wobei das Amyloid-Beta-Protein eine Fraktion von Amyloid-Beta-Protein ist.

14. Eine Verwendung nach Anspruch 11, wobei besagte Menge etwa 10⁻⁴ mg beträgt.

## Revendications

1. Composition pharmaceutique comprenant un véhicule pour une administration unique de protéine bêta amyloïde ou d'une fraction de celle-ci qui comprend une quantité jusqu'à 10⁻² mg de protéine bêta amyloïde ou de fraction de celle-ci et des ingrédients pharmaceutiques inertes.

2. Composition pharmaceutique selon la revendication 1, dans laquelle ladite quantité est comprise entre 10⁻⁸ et 10⁻² mg de protéine bêta amyloïde ou de fraction de celle-ci.

3. Composition pharmaceutique selon la revendication 2, dans laquelle ledit véhicule est une solution aqueuse qui est contenue à l'intérieur d'un conteneur inerte.

4. Composition pharmaceutique selon la revendication 2, dans laquelle ledit véhicule est un solide.

5. Composition pharmaceutique selon la revendication 4, sous la forme d'un comprimé sublingual.

6. Composition pharmaceutique selon la revendication 4, sous la forme d'un suppositoire.

7. Protéine bêta amyloïde ou fraction active de celle-ci utilisée pour soulager les symptômes d'états maladifs associés à l'accumulation anormale de la protéine bêta amyloïde et/ou à l'organisation moléculaire de celle-ci, des plaques névritiques ou des plaques amyloïdes associées au système nerveux central, et à des troubles histopathologiques, dans laquelle la protéine bêta amyloïde ou la fraction active de celle-ci est en une quantité jusqu'à 10⁻² mg par unité de dosage.

8. Protéine bêta amyloïde ou fraction de celle-ci selon la revendication 7, dans laquelle la protéine bêta amyloïde est sous la forme de la protéine bêta amyloïde elle-même.

9. Protéine bêta amyloïde ou fraction de celle-ci selon la revendication 7, dans laquelle la protéine bêta amyloïde est une fraction de la protéine bêta amyloïde.

10. Protéine bêta amyloïde ou fraction de celle-ci selon la revendication 7, dans laquelle ladite quantité est environ 10⁻⁴ mg.

11. Utilisation d'une protéine bêta amyloïde ou d'une fraction active de celle-ci pour la fabrication d'un médicament pour le soulagement d'états maladifs associés à l'accumulation anormale de la protéine bêta amyloïde et/ou à l'organisation moléculaire de celle-ci, des plaques névritiques ou des plaques amyloïdes associées au système nerveux central, et à des troubles histopathologiques, dans laquelle la protéine bêta amyloïde ou la fraction active de celle-ci est en une quantité jusqu'à 10⁻² mg par unité de dosage.

12. Utilisation selon la revendication 11, dans laquelle la protéine bêta amyloïde est sous la forme de la protéine bêta amyloïde elle-même.

13. Utilisation selon la revendication 11, dans laquelle le protéine bêta amyloïde est une fraction de la protéine bêta amyloïde.

14. Utilisation selon la revendication 11, dans laquelle ladite quantité est environ 10⁻⁴ mg.
